# EUROPÄISCHE PATENTSCHRIFT

(11) **EP 2 514 356 B1**
(45) Veröffentlichungstag und Bekanntmachung des Hinweises auf die Patenterteilung: **27.08.2014**
(21) Anmeldenummer: 12164725.9
(22) Anmeldetag: 19.04.2012
(51) Int. Cl.: A61B 1/002, A61B 1/04, A61B 1/055, A61B 1/00

(54) **Endoskop mit variabler Blickrichtung**
Endoscope with variable direction of view
Endoscope à direction d'observation variable

(30) Priorität: 20.04.2011 DE 102011007797
(43) Veröffentlichungstag der Anmeldung: 24.10.2012
(73) Patentinhaber: Henke-Sass, Wolf GmbH, 78532 Tuttlingen (DE)
(72) Erfinder: Rehe, Oliver, 78532 Tuttlingen (DE)
(74) Vertreter: Geyer, Fehners & Partner

(56) Entgegenhaltungen:
- DE-A1- 19 509 885
- DE-A1- 19 859 155
- DE-C1- 4 304 422
- US-A1- 2010 195 007

## Beschreibung

Die vorliegende Erfindung betrifft ein Endoskop mit variabler Blickrichtung mit den Merkmalen des Oberbegriffs des Anspruchs 1.

Solche Endoskope weisen häufig im distalen Ende des Endoskops einen schwenkbaren Spiegel oder ein schwenkbares Prisma auf, um die Blickrichtung zu ändern. Durch das Vorsehen des schwenkbaren Spiegels oder des schwenkbaren Prismas im distalen Ende des Endoskops ist ein erhöhter mechanischer Aufwand notwendig. Auch muß stets noch genügend Bauraum für das Verschwenken vorhanden sein und muß das gesamte Endoskop an sich einschließlich der Schwenkmechanik noch fertigbar sein, wodurch Grenzen für die Miniaturisierung gesetzt sind.

Dokument US 2010/0195007 A1 offenbart ein Endoskop mit variabler Blickrichtung.

Es ist ferner möglich, das Endoskop so auszubilden, daß die gesamte Endoskopspitze abwinkelbar ist, um die Blickrichtung zu ändern. In diesem Fall ist entweder ein flexibler Bildleiter oder ein in der Endoskopspitze eingebauter Bildsensor vorhanden. Jedoch sind solche Endoskope meist sehr empfindliche, wegen der notwendigen flexiblen Tülle für die Endoskopspitze, können nur sehr schwer in autoklavierbarer Ausführung hergestellt werden und sind sehr reparaturanfällig.

Ausgehend hiervon ist es daher Aufgabe der Erfindung, ein Endoskop mit variabler Blickrichtung zur Verfügung zu stellen, mit dem die eingangs beschriebenen Nachteile möglichst vollständig überwunden werden können.

Die Aufgabe wird erfindungsgemäß bei einem Endoskop der eingangs genannten Art dadurch gelöst, daß das erzeugte Bild größer ist als die Gesichtsfeldblende und im proximalen Bereich des Endoskops ein Bildmodul angeordnet ist, mit dem die relative Lage zwischen dem erzeugten Bild und der Gesichtsfeldblende veränderbar ist, um zur Änderung der Blickrichtung des Endoskops den nach der Gesichtsfeldblende sichtbaren Ausschnitt des erzeugten Bildes zu ändern.

Bei dem erfindungsgemäßen Endoskop wird die Änderung der Blickrichtung somit durch eine relative Lageänderung des erzeugten Bildes und der Gesichtsfeldblende im proximalen Bereich des Endoskops bewirkt, wodurch in vorteilhafter Weise am distalen Ende des Endoskops kein sich bewegendes Optikelement vorgesehen werden muß. Dadurch kann eine weitere Miniaturisierung des Endoskops und insbesondere des Endoskopschafts durchgeführt werden. Ferner kann die Herstellbarkeit sichergestellt werden, da keine sich bewegenden optischen Elemente im distalen Ende des Endoskops anzuordnen sind. Auch kann das Endoskop leicht autoklavierbar ausgebildet sein, da der Endoskopschaft als solcher als starrer Endoskopschaft ausgebildet werden kann.

Das erfindungsgemäße Endoskop ist insbesondere so ausgebildet, daß eine kontinuierliche bzw. stufenlose Änderung der relativen Lage zwischen dem erzeugten Bild und der Gesichtsfeldblende mittels dem Bildmodul möglich ist bzw. von diesem bewirkt wird. Dadurch wird eine kontinuierliche Änderung der Blickrichtung bewirkt. Man kann daher auch von einem "scannenden" Endoskop sprechen.

Unter einer kontinuierlichen bzw. stufenlosen Änderung wird hier insbesondere auch eine quasi kontinuierliche Änderung verstanden, die aufgrund einer mechanisch bedingten Mindestschrittweite vorliegt, die jedoch bevorzugt so klein wie möglich ist.

Die Abbildungsoptik erzeugt bevorzugt ein einzelnes Bild des Objektes, wobei das einzelne Bild zusammenhängend ist. Das Bild ist somit ausschließlich die Abbildung des Objektes in einem zusammenhängenden Bildwinkelbereich.

Bei dem erfindungsgemäßen Endoskop kann das erzeugte Bild in einer ersten Richtung größer sein als die Gesichtsfeldblende und kann die relative Lage zwischen dem erzeugten Bild und der Gesichtsfeldblende entlang der ersten Richtung veränderbar sein. Damit ist durch die Lageänderung in der ersten Richtung ein optisches Verschwenken der Blickrichtung in dieser Richtung möglich.

Insbesondere kann die Gesichtsfeldblende so angeordnet sein, daß sie entlang der ersten Richtung nicht verschiebbar ist.

Insbesondere kann mittels des Bildmoduls die relative Lage zwischen dem erzeugten Bild und der Gesichtsfeldblende nur entlang der ersten Richtung verändert werden.

Alternativ kann das Bildmodul so ausgebildet sein, daß die relative Lage zwischen dem erzeugten Bild und der Gesichtsfeldblende sowohl in der ersten Richtung als auch in einer dazu senkrechten zweiten Richtung veränderbar ist. In diesem Fall ist das erzeugte Bild bevorzugt auch in der zweiten Richtung größer als die Gesichtsfeldblende.

Das Bildmodul enthält bevorzugt zumindest ein bewegbares (bevorzugt stufenlos bewegbares) Optikelement, dessen Position die relative Lage zwischen dem erzeugten Bild und der Gesichtsfeldblende festlegt. Da das Bildmodul im proximalen Bereich des Endoskops angeordnet ist, ist hier mehr Platz bzw. Bauraum vorhanden, wodurch die Herstellbarkeit des Endoskops erleichtert ist.

Ferner kann das erfindungsgemäße Endoskop am proximalen Bereich ein Betätigungselement aufweisen, das mit dem zumindest einen bewegbaren Optikelement gekoppelt ist, so daß mittels des Betätigungselements die Position des zumindest einen bewegbaren Optikelementes einstellbar ist.

Bei dem Betätigungselement kann es sich beispielsweise um eine drehbar am Endoskopgriff gelagerte Hülse handeln. Es ist jedoch auch jede andere Ausbildung des Betätigungselementes möglich.

Die Kopplung zwischen Betätigungselement und dem zumindest einen bewegbaren Optikelement kann beispielsweise über Magnete erfolgen, so daß auch im proximalen Bereich das Endoskop für die Optik hermetisch dicht ausgebildet sein kann.

Das Bildmodul kann beispielsweise ein verschiebbares (bevorzugt stufenlos verschiebbares) Umlenkelement aufweisen. Ferner kann es ein Dove-Prisma aufweisen, das senkrecht zu seiner Grundfläche (bevorzugt stufenlos) verschiebbar ist.

Die Abbildungsoptik des erfindungsgemäßen Endoskops ist im Bereich des distalen Endes des Endoskops bevorzugt frei von bewegbaren Optikelementen. Damit wird eine einfache Miniaturisierung erreicht.

Die Abbildungsoptik kann am distalen Ende des Endoskops eine Linse aufweisen, die gleichzeitig eine Öffnung des Endoskopschafts am distalen Ende verschließt. Insbesondere kann sie diese Öffnung hermetisch dicht verschließen. Die Abbildung des Objektes und somit die Erzeugung des Bildes erfolgt über diese Linse.

Die Linse kann, in Draufsicht gesehen, länglich ausgebildet sein. Die Linse kann aus Glas oder Kunststoff hergestellt sein. Insbesondere ist die Linse aus Saphirglas hergestellt.

Die Linse kann ferner als Negativlinse ausgebildet sein.

Die Abbildungsoptik ist bevorzugt so ausgebildet, daß der Bildwinkel in einer ersten Richtung größer ist als in einer dazu senkrechten zweiten Richtung. So kann der Bildwinkel der ersten Richtung z.B. im Bereich von 130°-170° und bevorzugt im Bereich von 140°-160° liegen. Der Bildwinkel in der zweiten Richtung kann bevorzugt im Bereich von 50°-80°und insbesondere im Bereich von 60°-70° liegen. Es ist jedoch auch möglich, daß die Bildwinkel in beiden Richtungen gleichgroß sind.

Das erfindungsgemäße Endoskop kann neben der Linse am distalen Ende des Endoskops zumindest eine Beleuchtungsöffnung aufweisen. In dieser Beleuchtungsöffnung können beispielsweise Enden von Lichtleitfasern liegen. Es ist jedoch auch möglich, daß in diesen Beleuchtungsöffnungen direkt eine Lichtquelle, wie z.B. eine LED, angeordnet ist.

Das erfindungsgemäße Endoskop ist bevorzugt als Endoskop mit einem starren Endoskopschaft ausgebildet. Dies ist z.B. in der Sinuskopie von Vorteil, da der Operateur beim Einführen des Instrumentes in die Nase nach gerade ausschauen möchte und beim Erreichen des Operationsgebietes dann in eine andere Blickrichtung sehen will.

Natürlich kann das Endoskop auch zu ausgebildet sein, daß der Endoskopschaft zumindest in einem Abschnitt abwinkelbar ist.

Ferner kann das Endoskop im proximalen Bereich hinter der Gesichtsfeldblende ein Okular und/oder eine Schnittstelle für z.B. eine Videokamera aufweisen.

Bei dem erfindungsgemäßen Endoskop kann die Abbildungsoptik eine proximal letzte Linse aufweisen, die als Bildaufweitungslinse dient.

Ferner kann die Abbildungsoptik im distalen Endbereich des Endoskops ein Objektiv und daran proximal anschließend eine Bildübertragungsoptik (die beispielsweise Stablinsen enthält) aufweisen.

Das Bildmodul kann zumindest ein Optikelement enthalten, das zur Bildaufweitung, zur Bildübertragung und/oder zur Verzeichnungskorrektur dient.

Das Endoskop kann weitere, dem Fachmann bekannte Elementes aufweisen, die zum Betrieb des Endoskops notwendig sind.

Es wird ferner ein Endoskopieverfahren für ein Endoskop mit variabler Blickrichtung bereitgestellt, wobei das Endoskop einen Endoskopschaft und eine innerhalb des Endoskopschafts angeordnete Abbildungsoptik aufweist, die ein vor dem distalen Ende des Endoskops befindliches Objekt abbildet und ein Bild des Objektes im proximalen Bereich des Endoskopes erzeugt, in dem eine Gesichtsfeldblende angeordnet ist, wobei das erzeugte Bild in einer Größe erzeugt wird, die größer ist als die Gesichtsfeldblende, und die relative Lage zwischen dem erzeugten Bild und der Gesichtsfeldblende verändert wird, um den nach der Gesichtsfeldblende sichtbaren Ausschnitt des erzeugten Bildes und damit die Blickrichtung des Endoskopes zu ändern.

Insbesondere wird die Änderung der relativen Lage zwischen dem erzeugten Bild und der Gesichtsfeldblende stufenlos durchgeführt.

Des weiteren kann die Abbildungsoptik das Bild als einzelnes zusammenhängendes Bild erzeugen.

Das erfindungsgemäße Endoskopieverfahren kann noch weitere Schritte aufweisen, die im Zusammenhang mit dem erfindungsgemäßen Endoskop beschrieben sind.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer ersten Ausführungsform des erfindungsgemäßen Endoskops;
- Fig. 2: eine vergrößerte Schnittansicht des distalen Endes des Endoskopschafts von Fig. 1;
- Fig. 3: eine vergrößerte schematische Schnittansicht des proximalen Bereichs des Endoskops 1 von Fig. 1;
- Fig. 4: die Schnittansicht gemäß Fig. 3 mit geänderter Stellung des Prismas 13;
- Fig. 5: eine Darstellung zur Erläuterung der relativen Lage zwischen dem im proximalen Bereich erzeugten Bild B und der Gesichtsfeldblende 14;
- Fig. 6: eine weitere Darstellung zur Erläuterung der relativen Lage des im proximalen Bereich erzeugten Bildes B und der Gesichtsfeldblende 14;
- Fig.7: eine perspektivische vergrößerte Darstellung des distalen Endes des Endoskopschafts gemäß Fig. 1;
- Fig.8: eine schematische Schnittdarstellung des proximalen Bereichs des erfindungsgemäßen Endoskops gemäß einer weiteren Ausführungsform;
- Fig.9: die schematische Schnittdarstellung gemäß Fig. 8 mit einer geänderten Lage der Verschiebeeinheit 29 im Vergleich zu Fig. 8;
- Fig. 10: eine Darstellung der relativen Lage des im proximalen Bereich erzeugten Bildes B und der Gesichtsfeldblende 14;
- Fig. 11: eine weitere Darstellung der relativen Lage des erzeugten Bildes B und der Gesichtsfeldblende 14;
- Fig. 12: eine Darstellung der relativen Lage der Gesichtsfeldblende 14 zum erzeugten Bild im proximalen Bereich gemäß einer weiteren Ausführungsform, und
- Fig. 13: eine Darstellung der relativen Lage zwischen dem erzeugten Bild B und der Gesichtsfeldblende 14 zur Erläuterung der weiteren Ausführungsform.

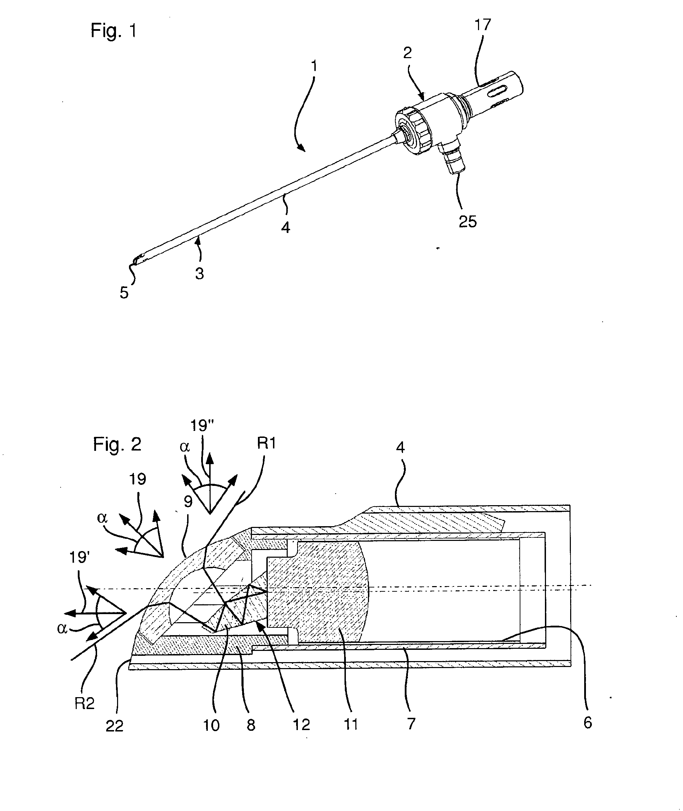

Bei der in Figur 1 gezeigten Ausführungsform ist das erfindungsgemäße Endoskop 1 als Endoskop 1 mit variabler Blickrichtung ausgebildet und weist einen Handgriff 2 sowie einen mit dem Handgriff 2 verbundenen Endoskopschaft 3 auf, dessen Mantelrohr 4 in Figur 1 sichtbar ist.

Wie insbesondere der vergrößerten Schnittdarstellung des distalen Endes 5 des Endoskopschaftes 3 in Figur 2 zu entnehmen ist, ist ein Optikrohr 6 vorgesehen, das in einem Innenrohr 7 sitzt. Das Innenrohr 7 seinerseits ist innerhalb des Mantelrohres 4 angeordnet. Am distalen Ende des Innenrohrs 7 ist ein Adapterstück 8 eingesetzt, dessen distales Ende als Fassung für eine Frontlinse 9 ausgebildet ist.

Die Frontlinse 9, die hier als Zerstreuungslinse aus Saphir ausgebildet ist, ist mit dem Adapterstück 8 verlötet, das seinerseits mit dem Innenrohr verlötet ist, so daß das distale Ende des Innenrohrs 7 und daher auch des Optikrohrs 6 hermetisch dicht verschlossen ist.

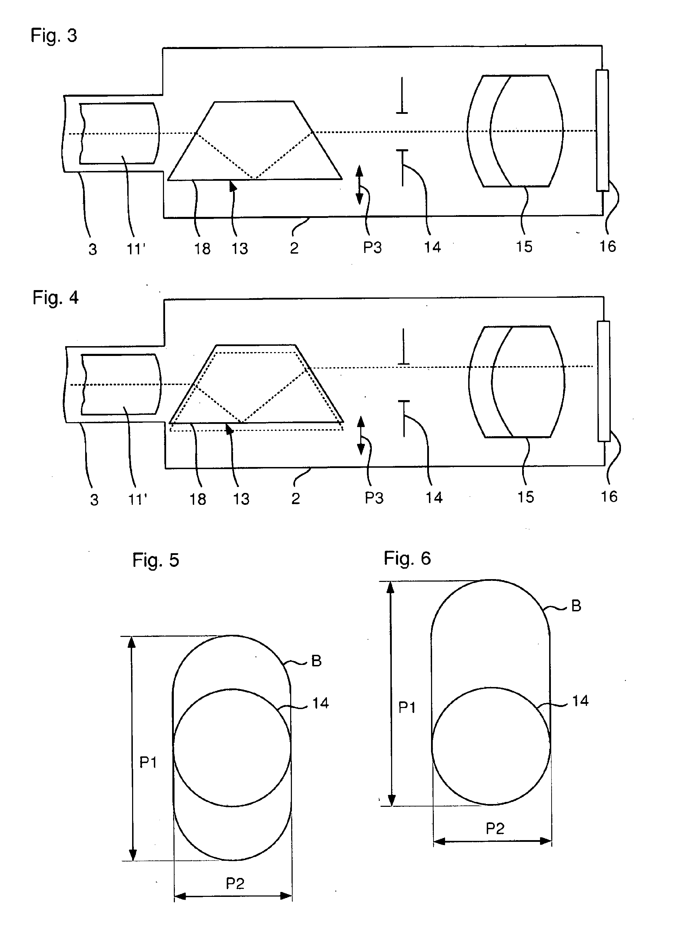

Im Bereich des Adapterstückes 8 ist ein Prismenelement 10 angeordnet, dem eine erste im Optikrohr 6 positionierte Objektivlinse 11 nachgeordnet ist. Im Optikrohr 6 sind weitere in Fig. 2 nicht gezeigte Objektivlinsen und Stablinsen vorhanden. In Fig. 3 ist die letzte Stablinse 11' gezeigt, die am proximalen Ende des Optikrohrs 6 sitzt.

Die Frontlinse 9, das Prismenelement 10 und die Objektiv- und Stablinsen 11, 11' sind Teil einer Abbildungsoptik 12, mit der ein Objekt vor dem distalen Ende 5 des Endoskops 1 so abgebildet wird, daß im proximalen Bereich (hier im Hangriff 2) des Endoskops 1 ein Bild des Objektes erzeugt wird.

Damit ein Benutzer das Objekt beobachten kann, ist im Handgriff 2 ein Dove-Prisma 13 angeordnet, dem eine Gesichtsfeldblende 14 folgt, hinter der ein Okular 15 angeordnet ist, um dem Benutzer durch ein Austrittsfenster 16 ein Ausschnitt des Bildes vergrößert darzubieten, wie nachfolgend im Detail beschrieben wird.

Die Abbildungsoptik 12 ist hier so ausgebildet, daß der aufnehmbare Bildwinkel in der Zeichenebene von Figur 2 (bzw. um eine senkrecht zur Zeichenebene verlaufenden Achse) sehr groß ist, beispielsweise 160°. Dies ist durch die eingezeichneten Randstrahlen R1, R2 des Sichtfeldes in der Zeichenebene von Figur 2 angedeutet. In einer Richtung senkrecht zur Zeichenebene von Figur 2 (bzw. um eine in der Zeichenebene liegende Achse, die hier mit der Längsrichtung des Endoskopschafts 3 einen Winkel von 45° einschließt) ist der aufnehmbare Bildwinkel kleiner, z.B. 70°, so daß insgesamt ein langgezogenes bzw. längliches Bild B am proximalen Ende des Endoskops (im Bereich der Gesichtsfeldblende 14) erzeugt wird, wie schematisch in Figur 5 dargestellt ist. Dabei entspricht die Länge des Doppelpfeils P1 dem größeren Bildwinkel (hier 160°), die Länge des Doppelpfeils P2 dem kleineren Bildwinkel (hier 70°).

In Figur 5 ist ferner die relative Position des Bildes B zur Gesichtsfeldblende 14 (die hier kreisförmig ist) dargestellt. Daraus ist ersichtlich, daß für einen Betrachter nur ein Ausschnitt a (Fig. 1) von 70° des langen Bildwinkels von 160° (Doppelpfeil P1) und der komplette kurze Bildwinkel von 70° (Doppelpfeil P2) mittels des Okulars 15 vergrößert dargestellt wird. Der lange Bildwinkel wird nachfolgend auch als erster Bildwinkel und der kurze Bildwinkel wird nachfolgend auch als zweiter Bildwinkel bezeichnet. Der in Figur 5 dargestellte Ausschnitt des ersten Bildwinkels entspricht einer ersten Blickrichtung 19 von 45° relativ zur Längsrichtung des Endoskopschafts 3, wie sie schematisch in Figur 2 eingezeichnet ist.

Das Dove-Prisma 13 im Handgriff 2 kann in Richtung des Doppelpfeils P3 (Figur 3) verschoben werden (senkrecht zur Grundfläche 18 des Dove-Prismas 13). Eine Verschiebung des Prismas 13 entlang der Richtung des Doppelpfeils P3 nach oben, wie in Figur 4 dargestellt ist, führt dazu, daß das Bild B relativ zu der feststehenden Gesichtsfeldblende 14 ebenfalls nach oben verschoben wird (Figur 6). In Figur 4 ist die ursprüngliche Position des Dove-Prismas 13 zur Verdeutlichung gestrichelt dargestellt.

Durch diese Lageänderung des Bildes B relativ zu der Gesichtsfeldblende 14, wird nun der Ausschnitt des Bildes B mittels des Okulars 15 vergrößert dargeboten, der der Blickrichtung 19' von 0° (Fig. 2) entspricht.

Ein Verschieben des Dove-Prismas 13 von der Stellung in Fig. 3 nach unten, würde zu einem Bildausschnitt führen, der z.B. eine Blickrichtung 19" von 90° (Figur 2) entspricht.

Bei sämtlichen einstellbaren Blickrichtungen beträgt der dargestellte Ausschnitt α des ersten Bildwinkels jeweils 70°, so daß mit den ersten Blickrichtungen von 0°-90° der gesamte erste Bildwinkel von -35°bis 125° darstellbar ist.

Ein Verschieben des Bildes B relativ zur Gesichtsfeldblende 14 führt somit zu einer Änderung der ersten Blickrichtung 19, 19', 19" für den Benutzer des Endoskops 1, wobei dazu erfindungsgemäß am distalen Ende des Endoskops 1 keinerlei bewegbares Optikelement vorgesehen werden muß. Dies führt zu dem Vorteil, daß der Querschnitt des Endoskopschafts bzw. der Durchmesser des Endoskopschafts 3 sehr klein gewählt werden kann (z.B. 4mm, 3,5mm, 3,2mm oder kleiner) und immer noch ein Endoskop 1 mit variabler Blickrichtung bereitgestellt ist.

Durch den großen ersten Bildwinkel P1 ist eine unverzerrte Abbildung des erzeugten Bildes B am proximalen Ende des Endoskops 1 schwierig zu erreichen. Da dem Benutzer jedoch nur immer ein gewisser Teilbereich (hier der Ausschnitt α) dieses ersten Bildwinkels aufgrund der Gesichtsfeldblende 14 dargeboten wird, ist es zur Erzielung einer gewünschten Abbildungsqualität ausreichend, wenn die Abbildungsoptik 12 so ausgelegt ist, daß innerhalb des durch die Gesichtsfeldblende 14 festgelegten sichtbaren Teilbereichs des ersten Bildwinkels die gewünschte Abbildungsqualität vorliegt.

Das bewegbare Element (Prisma 13) sitzt im proximalen Bereich des Endoskops 1 und hier im Handgriff 2. In diesem Bereich kann der Durchmesser größer gewählt werden, da dieser Bereich bei der Benutzung des Endoskops 1 nicht in die entsprechende Öffnung eingeführt wird.

Für die Bewegung des Dove-Prismas 13 kann am proximalen Ende des Endoskops 1 ein Halter (nicht gezeigt) vorgesehen sein, in dem das Dove-Prisma 13 sitzt, der über ein außen am Handgriff 2 angeordnetes Betätigungselement 17 (Figur 1) betätigt werden kann. Beispielsweise kann eine Magnet-Kopplung vorgesehen sein, um einen hermetisch dichten Innenraum im proximalen Ende für das Dove-Prisma 13, die Gesichtsfeldblende 14 und das Okular 15 bereitzustellen. In diesem Fall ist z.B. das Austrittsfenster 16 hermetisch dicht mit dem proximalen Ende des Endoskops 2 verbunden (z.B. durch Löten). Natürlich sind auch alle anderen Arten von mechanischer Kopplung zwischen Betätigungselement 17 und Halter für das Dove-Prisma 13 möglich, wie z.B. eine direkte mechanische Kopplung, falls eine hermetische Dichtheit im Bereich des Dove-Prismas nicht gewünscht ist.

Insbesondere kann das Betätigungselement 17 am Handgriff 2 drehbar und/oder in axialer Richtung verschiebbar gelagert sein, um die gewünschte Bewegung des Dove-Prismas 13 zu bewirken.

Das Betätigungselement 17, das Dove-Prisma 13 sowie deren mechanische Kopplung bilden ein Bildmodul, mit dem zur Einstellung der Blickrichtung 19, 19' des Endoskops 1 die relative Lage von erzeugtem Bild B und Gesichtsfeldblende 14 veränderbar und einstellbar ist.

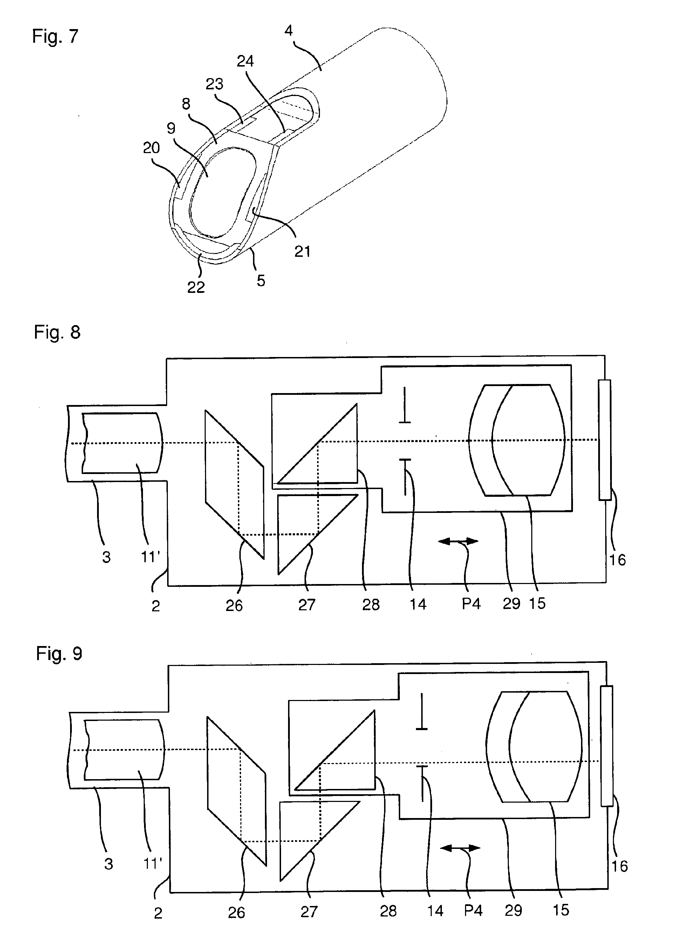

Durch das asymmetrische Bild B weist auch die Frontlinse 9, wie aus der perspektivischen Darstellung von Fig. 7 ersichtlich ist, eine längliche Form auf. Dies ist dahingehend von Vorteil, da seitlich neben der Frontlinse 9 Austrittsöffnungen 20 und 21, unterhalb der Frontlinse 9 eine Austrittsöffnung 22 und oberhalb der Frontlinse 9 zwei Austrittsöffnungen 23 und 24 vorgesehen werden können. Diese Austrittsöffnungen 20-24 sind z.B. Kanälen zugeordnet, die zwischen dem Innenrohr 7 und dem Mantelrohr 4 entlang der Längsrichtung des Endoskopfschafts 3 verlaufen. In diesen Kanälen können z.B. Lichtleitfasern (nicht gezeigt) angeordnet werden, die über einen Lichtleiteranschluß 25 (Figur 1) am Handgriff 2 mit Licht beaufschlagt werden können, so daß sie über die Austrittsöffnungen 20 bis 24 das Licht zur Beleuchtung eines vor dem distalen Ende 5 des Endoskops 1 liegenden Objektes abgeben können. Natürlich können in den Bereichen 20-24 auch andere Beleuchtungsmittel vorgesehen sein, wie z.B. LED.

Zwischen der in Figuren 3 und 4 gezeigten letzten Stablinse 11' und dem Prisma 13 kann eine nicht gezeigte Aufweitungsoptik (z.B. eine entsprechende Aufweitungslinse) angeordnet sein. Zusätzlich oder alternativ kann die letzte Stablinse 11' selbst als Aufweitungslinse bzw. Aufweitungsoptik ausgebildet sein. Damit wird das im proximalen Ende des Endoskops 1 erzeugte Bild des Objekts größer, wodurch die nachfolgenden mechanischen und optischen Teile auch größer ausgebildet werden können, was deren Herstellung vereinfacht. Da es sich hier um den proximalen Bereich des Endoskops 1 und somit um den Handgriff 2 handelt, ist eine Vergrößerung hier nicht nachteilig.

In Figur 8 und 9 ist eine Abwandlung des erfindungsgemäßen Endoskops gemäß Figuren 1 bis 7 gezeigt, wobei nachfolgend nur die Unterschiede beschrieben werden. So sind im Bereich des proximalen Endes statt dem Dove-Prisma 13 drei Prismen 26-28 vorgesehen. Von diesen drei Prismen sind die Prismen 26 und 27 feste Prismen, deren Lage nicht änderbar ist und ist das Prisma 28 ein bewegbares Prisma 28. Das Prisma 28 sitzt zusammen mit der Gesichtsfeldblende 14 und dem Okular 15 in einer Verschiebeeinheit 29, die relativ zu den festen Prismen 26 und 27 entlang der Längsrichtung des Endoskopschafts 2 verschiebbar ist, wie durch den Doppelpfeil P4 in Figuren 8 und 9 angedeutet ist. Dazu kann die Verschiebeeinheit 29 mit dem Betätigungselement 17 gekoppelt sein (z.B. in gleicher bzw. ähnlicher Weise wie die Kopplung von Betätigungselement 17 und Dove-Prisma 13 gemäß der Ausführungsform von Figuren 1 bis 7). In Abhängigkeit von der Verschiebeposition der Verschiebeeinheit 29 wird die relative Lage zwischen dem Bild B und der Gesichtsfeldblende 14 eingestellt. Daher kann in gleicher Weise wie bei der in Verbindung mit Figuren 1-7 beschriebenen Ausführungsform der entsprechende Ausschnitt des Bildes B und somit die erste Blickrichtung gewählt werden, die dem Betrachter über das Okular 15 dargeboten wird.

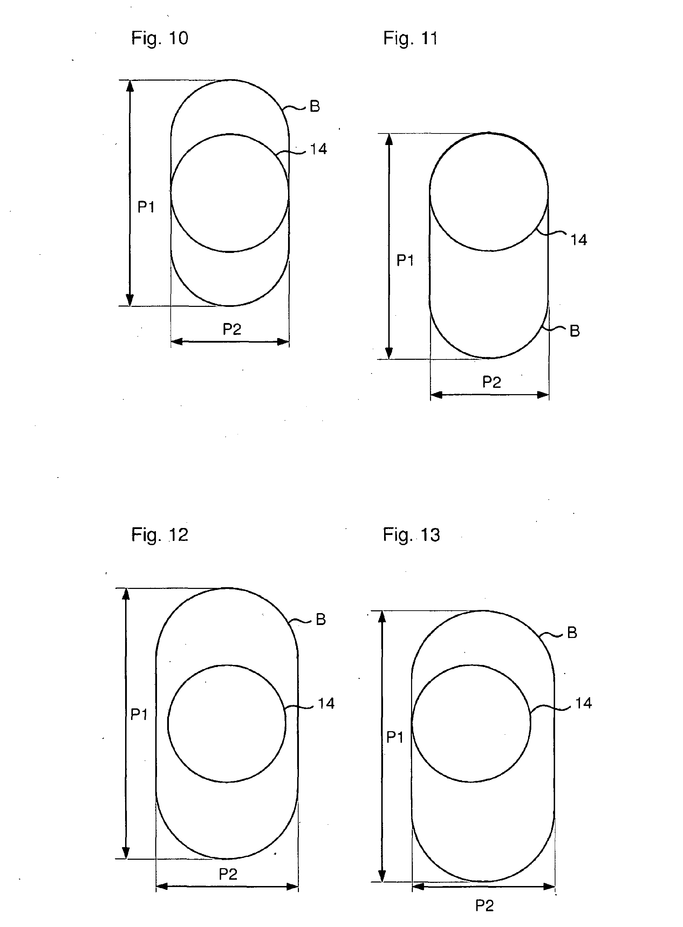

So kann z.B. die Verschiebeeinheit 29 im Vergleich zu ihrer Position in Figur 8 nach rechts verschoben werden, wie in Figur 9 gezeigt ist. Dies führt dann dazu, daß das Bild B entlang des ersten Bildwinkels P1 relativ zur Gesichtsfeldblende 14 nach unten verschoben ist, wie in Figuren 10 und 11 schematisch dargestellt ist, wobei Figur 10 die Lage des Bildes B relativ zur Gesichtsfeldblende 14 gemäß der Position der Verschiebeeinheit 29 von Figur 8 und Figur 11 die relative Lage des Bildes B zur Gesichtsfeldblende 14 gemäß der Position der Verschiebeeinheit 29 in Figur 9 zeigt. Die in Figur 11 vorliegende relative Lage zwischen dem Bild B und der Gesichtsfeldblende 14 entspricht einer ersten Blickrichtung 19" von 90°, wie sie in Figur 2 eingezeichnet ist.

Bei dieser Ausführungsform führt somit eine Bewegung von optischen Elementen im proximalen Ende des Endoskops 1 in Längsrichtung des Endoskopschafts 3 zu der gewünschten Änderung bzw. Einstellung der ersten Blickrichtung 19, 19', 19". Die Verschiebeeinheit 29, das Betätigungselement 17 und deren mechanische Kopplung bilden hier ein Bildmodul zur Einstellung der Blickrichtung.

Natürlich können mit den beschriebenen Ausführungsformen auch andere erste Blickrichtungen als die exemplarisch beschriebenen ersten Blickrichtungen 19, 19' und 19" eingestellt werden. Die konkrete erste Blickrichtung hängt stets von der relativen Lage des erzeugten Bildes B zur Gesichtsfeldblende 14 im proximalen Ende des Endoskops ab.

Das erzeugte Bild im Bereich der Gesichtsfeldblende 14 kann das dem Benutzer dargebotene Bild sein oder kann ein Zwischenbild sein, das zur Darstellung für den Benutzer verwendet wird.

Auch bei der in Verbindung mit Figuren 8 und 9 beschriebenen Ausführungsform kann in gleicher Weise wie bei der Ausführungsform gemäß Figuren 3 und 4 zwischen der letzten Stablinse 11' und dem Prisma 26 eine Aufweitungsoptik angeordnet sein. Ferner kann ebenfalls zusätzlich oder alternativ die letzte Stablinse 11' selbst als Aufweitungsoptik bzw. Aufweitungslinse ausgebildet sein. Des weiteren kann zusätzlich oder alternativ im proximalen Bereich des Endoskops bei allen beschriebenen Ausführungsformen zumindest ein weiteres optisches Element angeordnet werden, das z.B. zur Verbesserung der Bildqualität (beispielsweise Verzeichnungskorrektur) oder der Änderung der Bildgröße dient. Bei der in Verbindung mit Figuren 8 und 9 gezeigten Ausführungsform kann z.B. zwischen den beiden Prismen 26 und 27 eine Optik angeordnet werden, die dazu dient, daß die Bildübertragung mittels der Prismen 26 und 28 trotz der unter Umständen relativ langen Glaswege ohne Probleme möglich ist.

Bei dem Endoskop 1 gemäß der oben beschriebenen Ausführungsformen kann der Endoskopschaft 3 samt der gesamten Optikelemente im proximalen Ende des Endoskops 1 gegenüber dem Handgriff 2 drehbar gelagert sein, so daß durch eine Drehung des Endoskopschafts 3 eine Änderung der Blickrichtung in der zweiten Richtung, die der Richtung gemäß Doppelpfeil P2 entspricht, durchgeführt werden.

Bei der bisherigen Beschreibung ist davon ausgegangen worden, daß das Bild B nur entlang einer ersten Richtung (Doppelpfeil P1 in Figuren 5 und 6 sowie in Figuren 10 und 11) relativ zur Gesichtsfeldblende 14 verschoben wird und die Gesichtsfeldblende 14 in der zweiten Richtung (Doppelpfeil P2) das komplette Bild B abdeckt.

Natürlich kann das Endoskop 1 auch so ausgebildet sein, daß das Bild B auch in der zweiten Richtung (Doppelpfeil P2) eine größere Ausdehnung aufweist als die Gesichtsfeldblende 14, wie in Figur 12 schematisch angedeutet ist. Durch eine entsprechende Ausbildung des Bildmoduls kann das Bild B in beiden Richtungen relativ zur Gesichtsfeldblende 14 verschoben werden, was einer Änderung der ersten und zweiten Blickrichtung entspricht. Damit ist dann eine Änderung des dargestellten Ausschnitts des gesamten Bildwinkels der Abbildungsoptik 12 in unterschiedlichen Richtungen möglich, so daß der Benutzer die gewünschte Blickrichtung einstellen kann, ohne daß dazu bewegliche Teile am distalen Ende 5 des Endoskops 1 vorgesehen sein müssen.

So kann z.B. bei der Ausführungsform gemäß Figuren 3 und 4 hinter dem Dove-Prisma 13 und vor der Gesichtsfeldblende 14 ein zweites Dove-Prisma angeordnet sein, das gegenüber dem ersten Dove-Prisma 13 um 90° um die Längsachse des Endoskops 2 verdreht angeordnet ist. Das zweite Dove-Prisma kann in einer Richtung senkrecht zur Zeichenebene hin- und herbewegt werden, wobei diese Bewegung zu einer Verschiebung des Bildes B in der zweiten Blickrichtung (Doppelpfeil P2) führt. Damit kann die gewünschte Blickrichtung innerhalb des Bildes B festgelegt werden.

Die Gesichtsfeldblende 14 ist hier stets als Blende mit kreisförmiger Kontur beschrieben. Natürlich ist auch jede andere Konturform möglich, wie z.B. eine viereckige oder eine sonstige Polygonform.

Auch kann das erzeugte Bild B zwar länglich sein, es muß aber nicht diese Form aufweisen. Auch jede andere Form ist grundsätzlich möglich, wie z.B. eine Kreisform.

Bei dem erfindungsgemäßen Endoskop 1 kann die Abbildungsoptik 12 zwischen der Linse 9 und dem Prismenelement 10 noch zumindest ein weiteres Optikelement (wie z.B. eine Linse) aufweisen.

## Patentansprüche

1. Endoskop mit variabler Blickrichtung, das
einen Endoskopschaft (3) und
eine innerhalb des Endoskopschafts (3) angeordnete Abbildungsoptik (12) aufweist, die ein vor dem distalen Ende (5) des Endoskops (1) befindliches Objekt abbildet und ein Bild des Objekts im proximalen Bereich des Endoskops (1) erzeugt, in dem eine Gesichtsfeldblende (14) angeordnet ist,
**dadurch gekennzeichnet, daß**
das erzeugte Bild (B) größer ist als die Gesichtsfeldblende (14) und im proximalen Bereich des Endoskops (1) ein Bildmodul (17, 13; 17, 29) angeordnet ist, mit dem die relative Lage zwischen dem erzeugten Bild (B) und der Gesichtsfeldblende (14) veränderbar ist, um zur Änderung der Blickrichtung des Endoskops den nach der Gesichtsfeldblende (14) sichtbaren Ausschnitt des erzeugten Bildes (B) zu ändern,
wobei
das Bildmodul (17, 13; 17, 29) eine stufenlose Änderung der relativen Lage zwischen dem erzeugten Bild (B) und der Gesichtsfeldblende (14) bewirkt, und
die Abbildungsoptik (12) ein einzelnes zusammenhängendes Bild erzeugt.

2. Endoskop nach Anspruch 1, **dadurch gekennzeichnet, daß** das erzeugte Bild (B) in einer ersten Richtung (P1) größer ist als die Gesichtsfeldblende (14) und daß die relative Lage zwischen dem erzeugten Bild und der Gesichtsfeldblende (14) entlang der ersten Richtung veränderbar ist.

3. Endoskop nach Anspruch 2, **dadurch gekennzeichnet, daß** die Gesichtsfeldblende (14) entlang der ersten Richtung nicht verschiebbar ist.

4. Endoskop nach einem der obigen Ansprüche, **dadurch gekennzeichnet, daß** das Bildmodul (17, 13; 17, 29) zumindest ein bewegbares Optikelement (13, 28) enthält, dessen Position die relative Lage zwischen dem erzeugten Bild (B) und der Gesichtsfeldblende (14) festlegt.

5. Endoskop nach Anspruch 4, **dadurch gekennzeichnet, daß** das Endoskop am proximalen Bereich ein Betätigungselement (17) aufweist, das mit dem zumindest einen bewegbaren Optikelement (13, 28) gekoppelt ist, so daß mittels des Betätigungselementes (17) die Position des zumindest einen bewegbaren Optikelementes (13, 28) einstellbar ist.

6. Endoskop nach einem der obigen Ansprüche, **dadurch gekennzeichnet, daß** das Bildmodul (17, 29) ein verschiebbares Umlenkelement (28) aufweist.

7. Endoskop nach einem der obigen Ansprüche, **dadurch gekennzeichnet, daß** das Bildmodul (17, 13) ein Dove-Prisma (13) aufweist, das senkrecht zu seiner Grundfläche (18) verschiebbar ist.

8. Endoskop nach einem der obigen Ansprüche, **dadurch gekennzeichnet, daß** die Abbildungsoptik (12) im Bereich des distalen Endes (5) des Endoskops (1) frei ist von bewegbaren Optikelementen.

9. Endoskop nach einem der obigen Ansprüche, **dadurch gekennzeichnet, daß** die Abbildungsoptik (12) am distalen Ende des Endoskops eine Linse (9) aufweist, die gleichzeitig eine Öffnung des Endoskopschafts am distalen Ende verschließt.

10. Endoskop nach Anspruch 9, **dadurch gekennzeichnet, daß** die Linse (9), in Draufsicht gesehen, länglich ausgebildet ist.

11. Endoskop nach Anspruch 9 oder 10, **dadurch gekennzeichnet, daß** die Linse (9) als Negativlinse ausgebildet ist.

12. Endoskop nach einem der Ansprüche 9 bis 11, **dadurch gekennzeichnet, daß** neben der Linse (9) am distalen Ende des Endoskops (1) zumindest eine Beleuchtungsöffnung (20, 21, 22, 23, 24) vorhanden ist.

13. Endoskopieverfahren für ein Endoskop, wobei das Endoskop
einen Endoskopschaft und
eine innerhalb des Endoskopschafts angeordnete Abbildungsoptik aufweist, die ein vor dem distalen Ende des Endoskops befindliches Objekt abbildet und ein Bild des Objekts im proximalen Bereich des Endoskops erzeugt, in dem eine Gesichtsfeldblende angeordnet ist, **dadurch gekennzeichnet, daß**
das erzeugte Bild als einzelnes zusammenhängendes Bild größer erzeugt wird als die Gesichtsfeldblende und die relative Lage zwischen dem erzeugten Bild und der Gesichtsblende stufenlos veränderbar wird, um den nach der Gesichtsfeldblende sichtbaren Ausschnitt des erzeugten Bildes und damit die Blickrichtung des Endoskops zu ändern.

## Claims

1. Endoscope with a variable direction of view, which has
an endoscope shaft (3) and
imaging optics (12) arranged inside the endoscope shaft (3) which image an object located before the distal end (5) of the endoscope (1) and produce an image of the object in the proximal area of the endoscope (1) in which a field stop (14) is arranged,
**characterized in that**
the produced image (B) is larger than the field stop (14) and in the proximal area of the endoscope (1) an image module (17, 13; 17, 29) is arranged with which the relative position between the produced image (B) and the field stop (14) can be changed for changing the section of the produced image (B) visible after the field stop (14) in order to change the direction of view of the endoscope,
wherein the image module (17, 13; 17, 29) brings about a stepless change of the relative position between the produced image (B) and the field stop (14) and wherein the imaging optics (12) produce a single continuous image.

2. Endoscope according to claim 1, **characterized in that** the produced image (B) is larger in a first direction (P1) than the field stop (14) and **in that** the relative position between the produced image and the field stop (14) along the first direction can be changed.

3. Endoscope according to claim 2, **characterized in that** the field stop (14) cannot be displaced along the first direction.

4. Endoscope according to one of the above claims, **characterized in that** the image module (17, 13; 17, 29) contains at least one movable optical element (13, 28) whose position sets the relative position between the produced image (B) and the field stop (14).

5. Endoscope according to claim 4, **characterized in that** at the proximal area the endoscope has an actuating element (17) which is coupled to the at least one movable optical element (13, 28) with the result that the position of the at least one movable optical element (13, 28) can be set by means of the actuating element (17).

6. Endoscope according to one of the above claims, **characterized in that** the image module (17, 29) has a displaceable deflecting element (28).

7. Endoscope according to one of the above claims, **characterized in that** the image module (17, 13) has a Dove prism (13) which can be displaced perpendicular to its base surface (18).

8. Endoscope according to one of the previous claims, **characterized in that** the imaging optics (12) is free from movable optical elements in the area of the distal end (5) of the endoscope (1).

9. Endoscope according to one of the previous claims, **characterized in that** the imaging optics (12) have, at the distal end of the endoscope, a lens (9) which simultaneously seals an opening of the endoscope shaft at the distal end.

10. Endoscope according to claim 9, **characterized in that** the lens (9) is formed elongated in a top view.

11. Endoscope according to claim 9 or 10, **characterized in that** the lens (9) is formed as a negative lens.

12. Endoscope according to one of claims 9 to 11, **characterized in that** in addition to the lens (9) at the distal end of the endoscope (1) there is at least one illumination opening (20, 21, 22, 23, 24).

13. Endoscopy method for an endoscope, wherein the endoscope has
an endoscope shaft and
imaging optics arranged inside the endoscope shaft which image an object located before the distal end of the endoscope and produce an image of the object in the proximal area of the endoscope in which a field stop is arranged,
**characterized in that**
the produced image is produced as a single continuous image that is larger than the field stop and the relative position between the produced image and the field stop can be steplessly changed in order to change the section visible after the field stop of the produced image and thus the direction of view of the endoscope.

## Revendications

1. Endoscope à direction d'observation variable, qui présente une tige d'endoscope (3) et
une optique de visualisation (12) agencée à l'intérieur de la tige d'endoscope (3), qui figure un objet se trouvant devant l'extrémité distale (5) de l'endoscope (1 ) et produit une image de l'objet dans la zone proximale de l'endoscope (1), dans laquelle est agencé un diaphragme de champ visuel (14),
**caractérisé en ce que**
l'image produite (B) est plus grande que le diaphragme de champ visuel (14) et il est agencé dans la zone proximale de l'endoscope (1) un module d'image (17, 13 ; 17, 29) avec lequel le positionnement relatif entre l'image produite (B) et le diaphragme de champ visuel (14) peut être modifié afin de modifier la portion de l'image produite (B) visible après le diaphragme de champ visuel (14) de façon à modifier la direction d'observation de l'endoscope,
étant entendu que le module d'image (17, 13 ; 17, 29) provoque une variation continue du positionnement relatif entre l'image produite (B) et le diaphragme de champ visuel (14) et que l'optique de visualisation (12) produit une image unique d'un seul tenant.

2. Endoscope selon la revendication 1, **caractérisé en ce que** l'image produite (B), dans une première direction (P1), est plus grande que le diaphragme de champ visuel (14) et que le positionnement relatif entre l'image produite et le diaphragme de champ visuel (14) peut être modifié le long de la première direction.

3. Endoscope selon la revendication 2, **caractérisé en ce que** le diaphragme de champ visuel (14) ne peut pas coulisser le long de la première direction.

4. Endoscope selon l'une des revendications précédentes, **caractérisé en ce que** le module d'image (17, 13 ; 17, 29) contient au moins un élément optique mobile (13, 28), dont la position détermine le positionnement relatif entre l'image produite (B) et le diaphragme de champ visuel (14).

5. Endoscope selon la revendication 4, **caractérisé en ce que** l'endoscope présente dans la zone proximale un élément d'actionnement (17) qui est couplé avec l'au moins un élément optique mobile (13, 28) de telle sorte que la position de l'au moins un élément d'optique mobile (13, 28) peut être réglée au moyen de l'élément d'actionnement (17).

6. Endoscope selon l'une des revendications précédentes, **caractérisé en ce que** le module d'image (17, 29) présente un élément de déflection coulissant (28).

7. Endoscope selon l'une des revendications précédentes, **caractérisé en ce que** le module d'image (17, 13) présente un prisme de Dove (13) qui peut coulisser perpendiculairement à sa surface de base (18).

8. Endoscope selon l'une des revendications précédentes, **caractérisé en ce que** l'optique de visualisation (12) est dépourvue d'éléments optiques mobiles dans la zone de l'extrémité distale (5) de l'endoscope (1).

9. Endoscope selon l'une des revendications précédentes, **caractérisé en ce que** l'optique de visualisation (12) présente à l'extrémité distale de l'endoscope une lentille (9), qui obture en même temps une ouverture de la tige d'endoscope au niveau de l'extrémité distale.

10. Endoscope selon la revendication 9, **caractérisé en ce que** la lentille (9), vue du dessus, est réalisée dans une forme allongée.

11. Endoscope selon la revendication 9 ou 10, **caractérisé en ce que** la lentille (9) est réalisée comme lentille négative.

12. Endoscope selon l'une des revendications 9 à 11, **caractérisé en ce qu'**il est prévu à côté de la lentille (9), à l'extrémité distale de l'endoscope (1), au moins une ouverture d'éclairage (20, 21, 22, 23, 24).

13. Procédé d'endoscopie pour un endoscope, dans lequel l'endoscope présente une tige d'endoscope et
une optique de visualisation agencée à l'intérieur de la tige d'endoscope, qui figure un objet se trouvant devant l'extrémité distale de l'endoscope et produit une image de l'objet dans la zone proximale de l'endoscope, dans laquelle est agencé un diaphragme de champ visuel,
**caractérisé en ce que**
l'image produite est produite comme image individuelle d'un seul tenant plus grande que le diaphragme de champ visuel et le positionnement relatif entre l'image produite et le diaphragme de champ visuel peut être modifié de façon continue afin de modifier la portion de l'image produite visible après le diaphragme de champ visuel, et partant, la direction d'observation de l'endoscope.
